# EUROPÄISCHE PATENTSCHRIFT

(11) **EP 1 769 818 B1**
(45) Veröffentlichungstag und Bekanntmachung des Hinweises auf die Patenterteilung: **18.11.2009**
(21) Anmeldenummer: 06017209.5
(22) Anmeldetag: 18.08.2006
(51) Int. Cl.: A61M 15/00

(54) **Pulverinhalator**
Powder inhaler
Inhalateur de poudre

(30) Priorität: 29.09.2005 DE 102005046645
(43) Veröffentlichungstag der Anmeldung: 04.04.2007
(73) Patentinhaber: Braunform GmbH, 79353 Bahlingen (DE)
(72) Erfinder: Beller, Klaus-Dieter, Dr. med. Dipl.-Ing., 79341 Kenzingen (DE)
(74) Vertreter: Goy, Wolfgang

(56) Entgegenhaltungen:
- WO-A-00/64519
- WO-A-01/39823
- WO-A-03/061744
- WO-A-2004/060458
- WO-A2-03/061742
- US-B1- 6 752 147

## Beschreibung

Die Erfindung betrifft einen Pulverinhalator nach dem Oberbegriff des Anspruchs 1.

Der erfindungsgemäße treibgasfreie Pulverinhalator dient zur inhalativen Applikation von pulverförmigen, feststofflichen Arzneimitteln (oder anderen Mitteln). Die Pulverinhalatoren setzen dabei das Aerosol durch den Inhalationsvorgang frei, wobei die Energie für die Dispergierung durch den inspiratorischen Fluß gewonnen wird. Die pulverförmige Substanz ist dabei in einem Vorratsbehältnis enthalten. Je nach Art des Pulverinhalators wird der reine Wirkstoff eingesetzt oder der Wirkstoff mit einem Träger (unbedenklicher Hilfsstoff, meistens Laktose oder Glukose) für adhärierte Wirkstoffpartikel.

Mit dem durch WO 01/39823 bekannten Pulverinhalator ist es möglich, eine Pulverkombination zu inhalieren.

Der Erfindung liegt die **Aufgabe** zugrunde, einen Pulverinhalator zu schaffen, mit welchem wenigstens zwei unterschiedliche Pulvererfolgreicher verabreicht werden können.

Die technische **Lösung** ist gekennzeichnet durch die Merkmale im Kennzeichen des Anspruchs 1.

Dadurch ist ein Pulverinhalator geschaffen, mittels welchem mehrere unterschiedliche Pulver gleichzeitig verabreicht werden können. Die Grundidee besteht darin, in einem Pulverinhalator entsprechend der Anzahl der zu verabreichenden Pulver mehrere Vorratsbehältnisse vorzusehen. Somit werden die Pulver mit ihren Wirkstoffen getrennt gelagert. Auch werden die Pulver mit ihren Wirkstoffen getrennt dosiert und getrennt bereitgestellt. Die Pulver kommen erst beim Inhalieren im Inhalationsrohr miteinander in Kontakt, und zwar gerade so, wie wenn der Patient aus zwei Pulverinhalatoren gleichzeitig inhalieren würde.

Die Grundidee besteht darin, daß nur im inhalationsbereiten Zustand des Gerätes zur Führung des Luftstromes ein durchgängig ausgebildeter Luftkanal mit venturirohrartigem oder glattem Profil zur Verfügung steht, während im nicht inhalationsbereiten Zustand der Luftkanal gänzlich blockiert ist, so daß keinerlei Luft im Eingangsbereich und keinerlei pulvertragende Luft im Ausgangsbereich, nämlich im Inhalationsrohr, angesaugt werden kann. Somit ermöglicht ausschließlich nur zum Zeitpunkt der Anwendung ein entsprechend geformter Luftkanal einen optimalen Strömungsverlauf, und bei zwangsläufiger Synchronisation zwischen Deponieren der Pulver und Inhalation wird das portionierte pulverförmige Arzneimittel mitgenommen und auf der nachfolgenden Diffusorstrecke hinreichend vermischt und zerstäubt. Im geschlossenen Zustand hingegen ist die Eingangsöffnung sowie Austrittsöffnung gegenüber Feuchtigkeit und Staub automatisch verschlossen. Selbst bei akuter Atemnot kann der Patient noch die für die Pulver-Aerosolbehandlung mit dem Inhalator erforderliche Atmungsstärke aufbringen, da geradlinige Luftströmungskanäle realisierbar sind. Die synchron arbeitende Dosier- und Freigabeeinrichtungen ermöglichen die Minimierung der Bauteile und schließen das gesamte innere System des Gerätes bei Nichtgebrauch luft- und wasserdicht ab. Der Patient öffnet den Luftströmungskanal in dem Gerät mit einer einzigen Bewegung des Inhalationsrohres. Hierbei werden die Pulver deponiert und können sofort inhaliert werden. Nach der Inhalation schließt der Patient das System wiederum mit einer einzigen Bewegung durch Verschwenken des Inhalationsrohres. Hierbei werden aus den Vorratsbehältnisen die nächsten Dosen portioniert und das System luftdicht verschlossen. Durch das Verschwenksystem wird insgesamt eine zwangsweise richtige Handhabung für die Einnahmesicherheit gewährleistet. Ein Einwegeventil kann auf der Einstromseite für absolute Dichtigkeit sorgen. Da für die Inbetriebnahme des Pulverinhalators außer dem Inhalationsrohr keine weiteren Einrichtungen zu bewegen sind, bleibt aufgrund der Geradlinigkeit des Luftströmungskanals beim Inhalationsvorgang der größte Teil der Inspirationsenergie auch tatsächlich für die Inspiration erhalten. Da nur wenige mechanische Teile an dem Inhalator vorhanden sind und der Luftstrom nahezu geradlinig geführt wird, bleiben nur geringe Anteile der Pulver in der Vorrichtung, d.h. in dem Luftkanal haften. Dies erhöht die Dosiergenauigkeit. Mit der Mitnahme durch den Luftstrom werden die Pulver gleichmäßig vermischt, verteilt und deagglomeriert. Der Luftkanal hat bevorzugt die Form eines Venturirohres bzw. einer Lavaldüse. Zusätzlich werden beim Transport der Pulver und Mitnahme durch den Luftstrom gegebenenfalls größere Pulverpartikel durch schraubenförmige Lamellen zerschlagen und damit zerkleinert. In den Luftkanal können beliebig geformte mechanische Widerstände / Desagglomeratoren positioniert werden, um so den Luftkanal bedarfsgerecht sowie spezifikationsgerecht zu variieren. Der Luftkanal kann somit je nach Anforderung der Pulver im Atemzugswiderstand frei variiert werden. Die Ausführungen des Luftkanals können im Querschnitt rund oder oval sein und über die gesamte Länge den gleichen Querschnitt aufweisen. Der Einlaß und Auslaß kann beispielsweise trichterförmig sein. Durch das Venturirohr ist eine optimale Strömung und Beschleunigung des inspirierten Luftstromes einschließlich Mitnahme und Zerstäubung des pulverförmigen Arzneimittels gewährleistet. Insbesondere ist dadurch eine zwangsweise komplette Entleerung gewährleistet. Vorzugsweise sieht der Luftkanal ein Einwegventil vor, so daß der Patient das Pulver nur einatmen, nicht aber aus dem Inhalator hinausblasen kann. Die freie Gestaltung des Luftkanals ermöglicht die Anpassung des Inhalators an die verschiedensten inhalationsfähigen Arzneimittel. So können trockene pulverförmige Arzneimittel im Luftstrom in partikeldefinierter Größe dispergiert werden. Der erfindungsgemäße Inhalator ist insgesamt für einen längeren Gebrauch geeignet, da durch die Bauweise der gesamte Luftkanal mittels eines Pfeifenreinigers leicht zu reinigen ist und hygienische Probleme vermieden werden. Der Inhalator besteht bei Vollfunktion und einfachster Form aus wenigen Spritzgußteilen ohne die Verwendung von Federn und Hebeln.

Eine technische Realisierung des erfindungsgemäßen Pulverinhalators schlägt vor daß das Inhalationsrohr am mundfernen Ende drehbar gelagert ist. Dabei sind in der Nichtgebrauchsstellung des Inhalators sämtliche Öffnungen verschlossen, während sie in der Gebrauchsstellung des Inhalators im Sinne einer durchgängigen Luftströmung geöffnet sind. Vor allem ist dadurch der Luftstrom geradlinig ausgebildet und damit die Gefahr von Turbulenzen sowie Strömungswiderständen auf ein Minimum reduziert. Denn bei der Herstellung und Applikation von Pulveraerosolen spielt die Adhäsion und die Reibung eine bedeutende Rolle. Während der Applikation des Pulveraerosols müssen Adhäsion und Reibung zwischen Arzneimittel und Hilfsmittel überwunden werden. Aber gleichzeitig treten diese Kräfte zwischen den Pulverteilchen und den Pulverinhalatorflächen auf. Deswegen ist ein geradliniges Profil des Luftstromes von Vorteil. Es wird bei dem Pulverinhalator von Reservoirs für die Pulver ausgegangen, aus denen mehrere Dosiereinheiten portioniert werden können. Die Grundidee besteht darin, daß der Zylindermantel des Inhalationsrohres Durchgangsöffnungen in Form von Bohrungen aufweist, deren Höhe und Durchmesser das Dosiervolumen bestimmt und einer gegenüberliegenden Bohrung im zentralen Zylinderkörper entspricht. Im geschlossenen Zustand des Pulverinhalators liegen die Durchgangsöffnungen des Zylindermantels genau unterhalb der Auslaßöffnungen der Vorratsbehältnisse, so daß die Pulver dosiert werden. Im heruntergeklappten Zustand des Inhalationsrohres gelangen dann die Pulver in die Durchgangsbohrung im Zylinderkörper und damit exakt in den Luftkanal, so daß die Pulver bei Inspiration zunächst miteinander vermischt und inhaliert werden können. Die Vorratsbehältnisse können zur Erhöhung der Wirtschaftlichkeit auswechselbar sein.

Die Weiterbildung gemäß Anspruch 2 schlägt vor, daß ein gemeinsames Gehäuse vorgesehen ist, in welchem die Vorratsbehältnisse Wand an Wand liegen. Indem die Vorratsbehältnisse somit zueinander benachbart sind, besteht die Möglichkeit, daß die Auslaßöffnungen aus den Vorratsbehältnissen direkt nebeneinander liegen, wobei diese Auslaßöffnungen nach wie vor getrennt voneinander sind. Aus diesen Auslaßöffnungen können die Pulver ihren zugeordneten Dosiereinrichtungen zugeführt werden.

Gemäß der Weiterbildung in Anspruch 3 befindet sich im Lufteinlaß eine Verwirbelungseinrichtung für die Luft. Dies hat den Vorteil, daß durch die Verwirbelung der Vermischungsgrad der Pulver erhöht wird.

Dieser Vermischungsgrad wird weiterhin erhöht, wenn gemäß der Weiterbildung in Anspruch 4 sich in dem Inhalationsrohr und/oder in der Durchgangsbohrung ebenfalls eine Verwirbelungseinrichtung befindet.

Zwei Ausführungsbeispiele eines erfindungsgemäßen Pulverinhalators werden nachfolgend anhand der Zeichnungen beschrieben. In diesen zeigt:

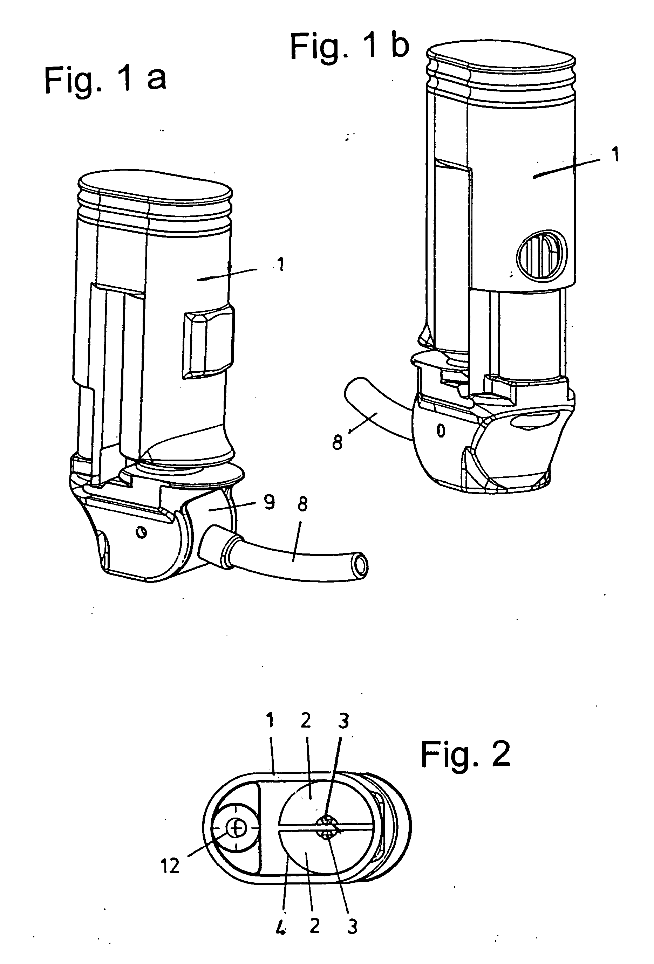

- Fig. 1a: eine erste Ausführungsform des Pulverinhalators in einer perspektivischen Ansicht von vorne für eine nasale Applikation;
- Fig. 1b: der Pulverinhalator in Fig. 1a in einer entsprechenden Darstellung von hinten;
- Fig. 2: einen Querschnitt durch den Pulverinhalator im Bereich der Vorratsbehältnisse;
- Fig. 3a: eine Längsschnittdarstellung durch den Pulverinhalator in der Längsmittelebene im Nichtgebrauchszustand;
- Fig. 3b: eine zu Fig. 3a senkrecht Schnittdarstellung;
- Fig. 4a: eine Darstellung entsprechend der in Fig. 3a in der Gebrauchsstellung;
- Fig. 4b: eine Darstellung entsprechend der in Fig. 3b in der Gebrauchsstellung;
- Fig. 5a: eine zweite Ausführungsform des Pulverinhalators mit einem Mundstück;
- Fig. 5b: der Pulverinhalator in Fig. 5a in einer entsprechenden Darstellung von hinten.

Der Pulverinhalator der ersten Ausführungsform weist ein Gehäuse 1 auf, in welchem sich zwei Vorratsbehältnisse 2 für unterschiedliche Pulver befinden. Diese beiden Vorratsbehältnisse 2 weisen an der untersten Stelle jeweils eine Auslaßöffnung 3 auf. Getrennt sind die beiden Vorratsbehältnisse 2 lediglich durch eine Wand 4 des Gehäuses 1, so daß die beiden Auslaßöffnungen 3 direkt nebeneinander liegen.

In dem Gehäuse 1 ist weiterhin ein Zylinderkörper 5 fest angeordnet. Dieser weist eine radiale Durchgangsbohrung 6 auf. Im Innern dieser Durchgangsbohrung 6 befindet sich eine flügelartige Verwirbelungseinrichtung 7.

Auf dem Zylinderkörper 5 ist ein Inhalationsrohr 8 drehbar gelagert. Zu diesem Zweck weist das hintere Ende dieses Inhalationsrohres 8 einen Zylindermantel 9 auf, mittels welchem die Verschwenklagerung auf dem Zylinderkörper 5 realisiert ist. Dieser Zylindermantel 9 weist einen Öffnung 10 auf, welche auf der Achse der Innenöffnung 11 des Inhalationsrohres 8 liegt.

Schließlich besitzt das Gehäuse 1 an der Rückseite einen Lufteinlaß 12, in welchem eine Verwirbelungseinrichtung 13 angeordnet ist.

### Die Funktionsweise ist wie folgt:

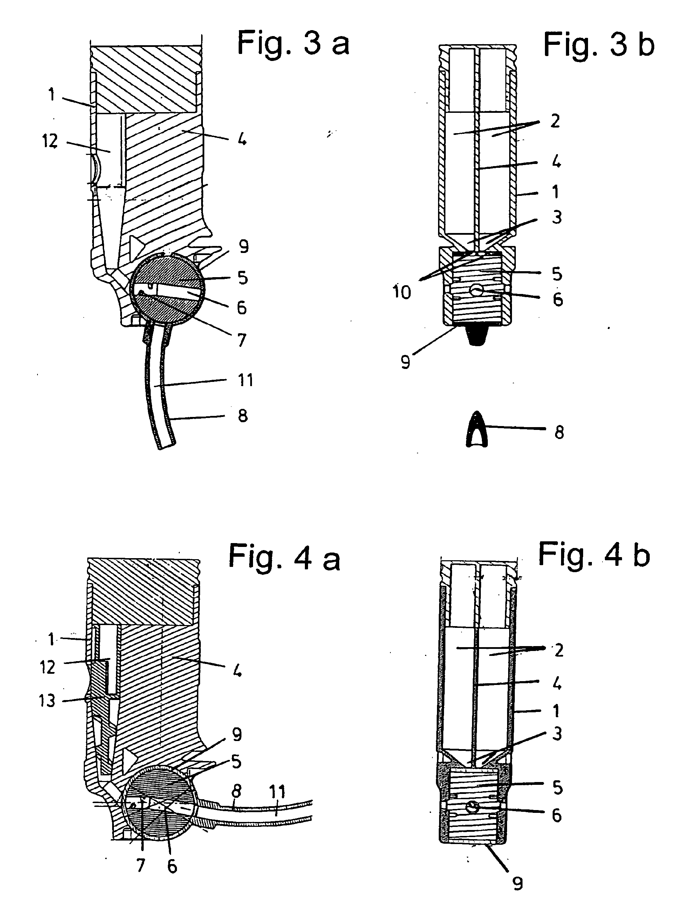

In der geschlossenen Nichtgebrauchsstellung (Fig. 3a und 3b) befinden sich die beiden Öffnungen 10 des Zylindermantels 9 unterhalb der beiden Auslaßöffnungen 3 der Vorratsbehältnisse 2. Die in den Vorratsbehältnissen 2 befindlichen Pulver rieseln unabhängig voneinander in die Öffnungen 10. Diese definieren ein Volumen und damit eine Dosiereinheit. In dieser Stellung ist die Durchgangsbohrung 6 des Zylinderkörpers 5 durch den Zylindermantel 9 abgedichtet geschlossen.

Zum Überführen des Pulverinhalators in die Gebrauchsstellung (Fig. 4a und 4b) wird das Inhalationsrohr 8 nach oben verschwenkt. Dadurch gelangen die beiden Öffnungen 10 des Zylindermantels 9 mit den darin befindlichen Dosiereinheiten für die beiden Pulver in den Bereich der Durchgangsbohrung 6 des Zylinderkörpers 5. Die Innenöffnung 11 des Inhalationsrohres 8, die Durchgangsbohrung 6 des Zylinderkörpers 5, die beiden Öffnungen 10 im Zylindermantel 9 sowie der Lufteinlaß 12 des Gehäuses 1 definieren dabei einen miteinander verbundenen, durchgängigen sowie abgedichteten Durchgangskanal.

Durch die Inhalation durch den Benutzer werden die beiden Pulversorten aus ihren Öffnungen 10 mitgerissen, miteinander vermischt und letztendlich vom Patienten eingeatmet.

Nach der Inhalation wird das Inhalationsrohr 8 wieder nach unten verschwenkt (Fig. 3a und 3b), so daß eine neue Dosis an Pulver aus den Vorratsbehältnissen 2 durch die Auslaßöffnungen 3 hindurch in die Öffnungen 10 des Zylindermantels 9 gelangen.

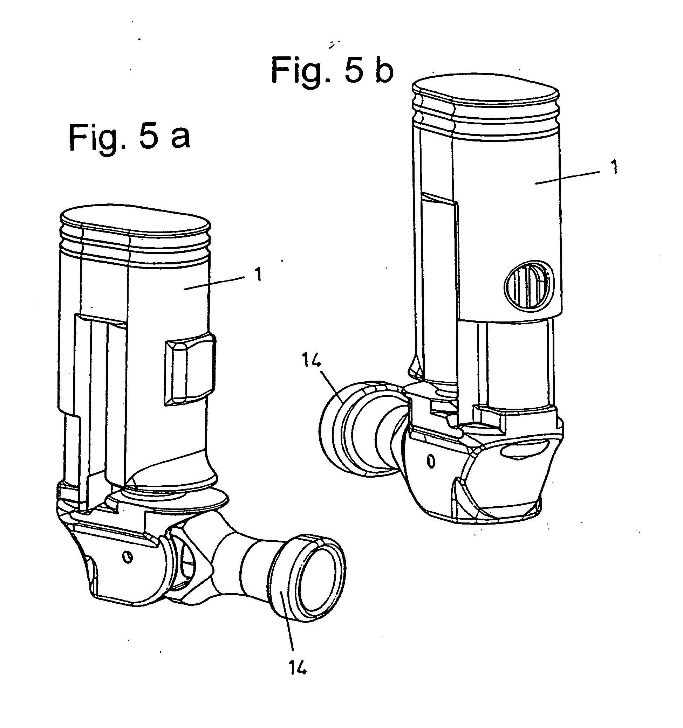

Bei dem dargestellten Ausführungsbeispiel ist das Inhalationsrohr 8 als Nasenrohr für eine nasale Applikation ausgebildet. Das Inhalationsrohr 8 kann jedoch gleichermaßen auch als Mundrohr 14 ausgebildet sein, wie dies in den Fig. 5a und 5b dargestellt ist. Der konstruktive Aufbau ist dabei vom Grundprinzip her der gleiche wie bei der nasalen Applikation.

### Bezugszeichenliste

- 1: Gehäuse
- 2: Vorratsbehältnisse
- 3: Auslaßöffnungen
- 4: Wand
- 5: Zylinderkörper
- 6: Durchgangsbohrung
- 7: Verwirbelungseinrichtung
- 8: Inhalationsrohr
- 9: Zylindermantel
- 10: Öffnungen
- 11: Innenöffnung
- 12: Lufteinlaß
- 13: Verwirbelungseinrichtung
- 14: Mundrohr

## Patentansprüche

1. Pulverinhalator
mit wenigstens zwei Vorratsbehältnissen (2), in welchen unterschiedliche Pulver getrennt voneinander in mehreren Dosiereinheiten bevorratet sind,
mit jeweils einer Auslaßöffnung (3) an der Unterseite der Vorratsbehältnisse (2) sowie mit einer drehbaren Dosiereinrichtung, mittels welcher die Pulver aus den Vorratsbehältnissen (2) getrennt voneinander dosierbar sind,
wobei in einer Zylindermantelflache dieser Dosiereinrichtung zu den Auslaßöffnungen (3) korrespondierende Öffnungen (10) ausgebildet sind und die Dosiereinrichtungen definieren,
**dadurch gekennzeichnet,**
**daß** ein für beide Pulver gemeinsames Inhalationsrohr (8) zum Inhalieren der Pulver vorgesehen ist, wobei während des Inhalierens ein durchgängiger Luftstrom die Pulver mitreißt und miteinander verwischt,
**daß** das Inhalationsrohr (8) mit seinem hinteren Ende mittels eines angeformten Zylindermantels (9) der Dosiereinrichtung auf einem feststehenden Zylinderkörper (5) derart verschwenkbar gelagert ist,
**daß** zum einen in einer Nichtgebrauchsstellung des Inhalationsrohres (8) die Pulver in den Vorratsbehältnissen (2) luftdicht abgeschlossen sind und
**daß** zum anderen beim Verschwenken des Inhalationsrohres (8) in die Gebrauchsstellung die Pulver in den Bereich des durchgängigen Luftstromes in dem Inhalationsrohr (8) gelangen,
**daß** der Zylinderkörper (5) eine radiale Durchgangsbohrung (6) aufweist,
**daß** der Zylindermantel (9) in der axialen Verlängerung des Inhalationsrohres (8) entsprechend der Anzahl der Vorratsbehältnisse (2) getrennte, durchgehende Öffnungen (10) aufweist,
**daß** in der Nichtgebrauchsstellung des Inhalationsrohres (8) sich die Innenöffnung (11) des Inhalationsrohres (8) und die Öffnungen (10) des Zylindermantels (9) nicht im Bereich der Durchgangsbohrung (6) des Zylinderkörpers (5) befinden, wobei sich stattdessen die Öffnungen (10) des Zylindermantels (9) in dieser Stellung unterhalb der korrespondierenden Auslaßöffnungen (3) der Vorratsbehältnisse (2) befinden, und
**daß** in der Gebrauchsstellung des Inhalationsrohres (8) sich zum einen die Innenöffnung (11) des Inhalationsrohres (8) und zum anderen die Öffnungen (10) des Zylindermantels (9) mit den darin befindlichen Pulvern in Verlängerung der Durchgangsbohrung (6) des Zylinderkörpers (5) sowie der Mündung eines Lufteinlasses (12) befinden.

2. Pulverinhalator nach dem vorhergehenden Anspruch,
**dadurch gekennzeichnet,**
**daß** die Vorratsbehältnisse (2) Wand (4) an Wand (4) liegen und direkt nebeneinanderliegende Auslaßöffnungen (3) zu den Dosiereinrichtungen aufweisen.

3. Pulverinhalator nach einem der vorhergehenden Ansprüche,
**dadurch gekennzeichnet,**
**daß** sich im Lufteinlaß (12) eine Verwirbelungseinrichtung (13) für die Luft befindet.

4. Pulverinhalator nach einem der vorhergehenden Ansprüche,
**dadurch gekennzeichnet,**
**daß** sich im Inhalationsrohr (8) und/oder in der Durchbohrung (6) eine Verwirbelungseinrichtung (7) befindet.

## Claims

1. Powder inhaler with at least two reservoir containers (2), in which different powders are stored separately from one another in a plurality of metering units, with a respective outlet opening (3) on the lower side of the reservoir container (2) and with a rotatable metering device, by means of which the powders can be metered separately from one another from the reservoir containers (2), wherein openings (10) corresponding with the outlet openings (3) are configured in a lateral cylinder surface of this metering device and define the metering devices, **characterised in that** an inhalation tube (8) common to the two powders is provided for the inhalation of the powders, a continuous airflow entraining the powders and mixing them with one another during the inhalation, **in that** the inhalation tube (8) is pivotably mounted by its rear end by means of a moulded-on lateral cylinder surface (9) of the metering device on a fixed cylinder body (5), in such a way that, on the one hand, the powders are sealed in an air-tight manner in the reservoir containers (2) in a non-use position of the inhalation tube (8) and **in that**, on the other hand, when the inhalation tube (8) is pivoted into the use position, the powders arrive in the region of the continuous airflow in the inhalation tube (8), **in that** the cylinder body (5) has a radial through-bore (6), **in that** the lateral cylinder surface (9) has separate, continuous openings (10) in the axial extension of the inhalation tube (8) in accordance with the number of reservoir containers (2), **in that**, in the non-use position of the inhalation tube (8), the inner opening (11) of the inhalation tube (8) and the openings (10) of the lateral cylinder surface (9) are not located in the region of the through-bore (6) of the cylinder body (5), the openings (10) of the lateral cylinder surface (9) in this position instead being located below the corresponding outlet openings (3) of the reservoir container (2), and **in that**, in the use position of the inhalation tube (8), on the one hand, the inner opening (11) of the inhalation tube (8) and, on the other hand, the openings (10) of the lateral cylinder surface (9) with the powders located therein are located in the extension of the through-bore (6) of the cylinder body (5) and the mouth of an air inlet (12).

2. Powder inhaler according to the preceding claim, **characterised in that** the reservoir containers (2) lie wall (4) to wall (4) and have outlet openings (3), which are located directly next to one another, to the metering devices.

3. Powder inhaler according to any one of the preceding claims, **characterised in that** a swirling device (13) for the air is located in the air inlet (12).

4. Powder inhaler according to any one of the preceding claims, **characterised in that** a swirling device (7) is located in the inhalation tube (8) and/or in the through-bore (6).

## Revendications

1. Inhalateur de poudre comprenant
au moins deux récipients de réserve (2) dans lesquels des poudres différentes sont stockées séparément l'une de l'autre dans plusieurs unités de dosage,
un orifice respectif de sortie (3), à la face inférieure desdits récipients de réserve (2),
ainsi qu'un dispositif rotatif de dosage au moyen duquel les poudres peuvent être dosées séparément l'une de l'autre à partir desdits récipients de réserve (2),
sachant que des orifices (10), pratiqués dans une surface d'enveloppe cylindrique de ce dispositif de dosage, concordent avec les orifices de sortie (3) et définissent les dispositifs de dosage,
**caractérisé**
**par le fait qu'**un tube inhalateur (8), commun aux deux poudres, est prévu pour l'inhalation desdites poudres, un courant d'air continu entraînant lesdites poudres et les mélangeant l'une à l'autre au cours de l'inhalation ;
**par le fait que** ledit tube inhalateur (8) est monté à pivotement sur un corps cylindrique fixe (5) par son extrémité postérieure, au moyen d'une enveloppe cylindrique (9) du dispositif de dosage solidarisée par moulage, de façon telle
que d'une part, dans une position de non-utilisation dudit tube inhalateur (8), les poudres soient confinées dans les récipients de réserve (2) avec étanchéité à l'air, et
que d'autre part, lors du pivotement dudit tube inhalateur (8) jusqu'à la position d'utilisation, lesdites poudres gagnent la région du courant d'air continu dans ledit tube inhalateur (8) ;
**par le fait que** le corps cylindrique (5) présente un canal radial de passage (6) ;
**par le fait que** l'enveloppe cylindrique (9) présente, dans le prolongement axial du tube inhalateur (8), des orifices distincts ininterrompus (10) dont le nombre correspond à celui des récipients de réserve (2) ;
**par le fait que**, dans la position de non-utilisation du tube inhalateur (8), l'orifice intérieur (11) dudit tube inhalateur (8) et les orifices (10) de l'enveloppe cylindrique (9) ne se trouvent pas dans la région du canal de passage (6) du corps cylindrique (5), lesdits orifices (10) de ladite enveloppe cylindrique (9) étant en revanche situés, dans cette position, au-dessous des orifices complémentaires de sortie (3) des récipients de réserve (2) ; et
**par le fait que**, dans la position d'utilisation du tube inhalateur (8), d'une part l'orifice intérieur (11) dudit tube inhalateur (8) et, d'autre part, les orifices (10) de l'enveloppe cylindrique (9) et les poudres qu'ils renferment se trouvent dans le prolongement du canal de passage (6) du corps cylindrique (5), ainsi que de l'embouchure d'une admission d'air (12).

2. Inhalateur de poudre selon la revendication précédente,
**caractérisé par le fait**
**que** les récipients de réserve (2) sont en applique paroi (4) contre paroi (4) et comportent des orifices de sortie (3) en juxtaposition directe, menant aux dispositifs de dosage.

3. Inhalateur de poudre selon l'une des revendications précédentes,
**caractérisé par le fait**
**qu'**un dispositif (13) générateur de tourbillons, destiné à l'air, se trouve dans l'admission d'air (12).

4. Inhalateur de poudre selon l'une des revendications précédentes,
**caractérisé par le fait**
**qu'**un dispositif (7) générateur de tourbillons se trouve dans le tube inhalateur (8) et/ou dans le canal de passage (6).
